# EUROPÄISCHE PATENTANMELDUNG

(11) **EP 1 138 339 A2**
(43) Veröffentlichungstag der Anmeldung: **04.10.2001**
(21) Anmeldenummer: 01106230.4
(22) Anmeldetag: 14.03.2001
(51) Int. Cl.: A61M 11/04

(54) **Vorrichtung zur Freisetzung ätherischer Wirkstoffe aus Heilsalben**

(30) Priorität: 23.03.2000 DE 10014505
(71) Anmelder: Badewien, Reinhard, 26802 Moormerland (DE)
(72) Erfinder: Badewien, Reinhard, 26802 Moormerland (DE)

(57) **Zusammenfassung**

Eine Vorrichtung zum Freisetzen ätherischer Wirkstoffe aus Heilsalben umfaßt einen Wassertopf, eine Beheizeinrichtung für das im Wassertopf aufgenommene Wasser sowie einen auf der Wasseroberfläche schwimmenden Schwimmnapf, in den Heilsalbe eingebbar ist. Eine Führungseinrichtung zum Nachführen des Schwimmnapfes bei abnehmendem Wasserstand im Wassertopf ist vorgesehen. Auf dem Wasser schwimmt ebenfalls eine über den Schwimmnapf stülpbare Verschlußglocke, die so weit ist, daß zwischen Schwimmnapf und Verschlußglocke ein Spalt oder Zwischenraum vorbestimmter Größe verbleibt. Der untere Rand der Verschlußglocke ist in das Wasser getaucht. Dadurch wird eine Freisetzung der Dämpfe aus der Heilsalbe an die Außenluft nur über das Wasser ermöglicht, wobei dabei eine Ausfilterung unerwünschter Bestandteile der Salbe bewirkt wird.

## Beschreibung

Die Erfindung bezieht sich auf eine Vorrichtung zur Freisetzung ätherischer Wirkstoffe aus Heilsalben; mit einem Wassertopf, einer Beheizeinrichtung für das im Wassertopf aufgenommene Wasser, mit einem eine vorbestimmte Menge der Heilsalbe aufnehmenden, auf der freien Oberfläche des Wassers im Wassertopf schwimmenden Schwimmnapf, und mit einer Führungseinrichtung zum Nachführen des Schwimmnapfes bei abnehmendem Wasserstand im Wassertopf.

Zur Behandlung entzündlicher Erkrankungen der Atemwege ist es bekannt, Salben zu verwenden, die zum Beispiel Eukalyptusöl, Fichtennadelöl, Pfefferminzöl und dergleichen ätherische Wirkstoffe bzw. Wirkstoffkombinationen enthalten. Salben können in heißes Wasser eingegeben werden, so daß die dann freigesetzten, mit den ätherischen Wirkstoffen angereicherten Dämpfe inhalierbar sind.

Jede Salbe enthält neben den medizinisch wirksamen Stoffen in nicht unerheblichen Anteilen auch weitere Beimengungen, wie z.B. Fette, Alkohole, Emulgatoren, Stabilisatoren und dergleichen Chemikalien. Deren Freisetzung und Anteil an den inhalierbaren Dämpfen ist an und für sich unerwünscht, denn sie können, insbesondere mit Luft, zu Verbindungen reagieren, die als unangenehm riechend empfunden werden.

Der Erfindung liegt die Aufgabe zugrunde, eine Vorrichtung zu schaffen, die aus Salben allein die ätherischen, medizinisch wirksamen Stoffe freisetzt, nicht jedoch die sonstigen Stoffe und Beimengungen der jeweils verwendeten Salbe.

Diese Aufgabe ist erfindungsgemäß dadurch gelöst, daß eine über den Schwimmnapf stülpbare Verschlußglocke vorgesehen ist, die um ein vorbestimmtes Maß weiter als der übergriffene Schwimmnapf ist und deren unterer Glockenrand bei auf dem Wasser schwimmendem Schwimmnapf in das Wasser eingetaucht ist.

Die Verschlußglocke des Schwimmnapfes, der mit einer vorbestimmten Portion Salbe füllbar ist, hat den Vorteil, daß sie eine Ausfilterung der chemischen Zusatzstoffe, die neben den medizinischen Wirkstoffen aus der Salbe freigesetzt werden, bewirkt. Die über den auf dem Wasser im Wassertopf schwimmenden Schwimmnapf gestülpte Verschlußglocke schwimmt ebenfalls auf dem Wasser, da ihr unterer Rand in das Wasser eintaucht, wobei sie von dem in ihr enthaltenen Luftpolster getragen wird.

Durch die Beheizeinrichtung, z.B. eine elektrische Heizplatte, wird das im Wassertopf enthaltene Wasser erwärmt und durch Wärmeübertragung letztlich auch die im Schwimmnapf enthaltene Salbe. Aus der Salbe dunsten Dämpfe ihrer Inhaltsstoffe aus. Da die Verschlußglocke weiter ist als der von ihr übergriffene Schwimmnapf, ist in dem Zwischenraum bzw. Spalt zwischen der Innenwand der Verschlußglocke und der Außenwand des Schwimmnapfes eine freie Oberfläche des Wassers vorbestimmter Größenordnung vorhanden. Im Zwischenraum über dem Wasser stehende Luft, die mit aus der Salbe ausgedünsteten Dämpfen angereichert ist, ermöglicht es, daß das Wasser im Zwischenraum die Dämpfe aufnimmt. Aufgrund der sich im Wasser ausbildenden, durch Beheizung entstehenden und aufrecht erhaltenen Thermik, bzw. umwälzenden Strömung, gelangen die nunmehr im Wasser enthaltenen Inhaltsstoffe der Salbe auch in Bereiche der außerhalb der Verschlußglocke befindlichen freien Wasseroberfläche. Dort können sie in die über der Wasseroberfläche im Wassertopf anstehende Außenluft übertreten.

Es hat sich gezeigt, daß nur die medizinisch wirksamen Inhaltsstoffe wieder aus dem Wasser freigesetzt werden. Die evt1. im Wasser ebenfalls enthaltenen Zusatzstoffe, wie Emulgatoren, Stabilisatoren und dergl. Chemikalien bleiben im Wasser hängen.

Dabei ist es des weiteren äußerst vorteilhaft, daß das Wasser umso mehr an ätherischen Inhaltsstoffen der aus der Salbe freigesetzten Dämpfe annimmt, desto breiter der Zwischenraum bzw. der Spalt zwischen Schwimmnapf und Verschlußglocke ist. Je größer also die zwischen Schwimmnapf und Verschlußglocke befindliche Wasseroberfläche ist, desto höher wird die Konzentration der medizinisch wirksamen Stoffe in der Außenluft. Diese Außenluft ist insbesondere die Luft in einem Raum, in dem sich eine an Atemwegserkrankungen leidende Person aufhält, z.B. ein Schlafraum.

Mit unterschiedlich weiten Verschlußglocken ist es somit möglich, eine Konzentration der Wirkstoffe in der einzuatmenden Luft zu variieren. Bei einem Zwischenraum von z.B. 1,5mm Breite stellt sich eine angenehme, milde Konzentration in einem Raum ein. Bei einem beispielsweise 5mm breiten Zwischenraum ist die Konzentration bedeutend stärker. Die erfindungsgemäße Vorrichtung kann für derartige Variationen vorzugsweise mit einem Satz verschieden weiter Verschlußglocken ausgerüstet bzw. bereitgestellt werden.

Eine Alternative oder auch zusätzliche Dosiermöglichkeit ist durch Variation der Menge der Salbe möglich, die in den Schwimmnapf eingegeben wird. Um die Handhabung bei der Eingabe einzelner kleinerer Salbenportionen zu erleichtern, ist der Schwimmnapf mit voneinander getrennten Portions-Kammern ausgerüstet.

Sowohl die Verschlußglocke als auch der Schwimmnapf weisen mit der Führungseinrichtung in Wirkverbindung bringbare Führungselemente bzw. Führungsorgane auf. Diese bewirken, daß sowohl Schwimmnapf als auch Verschlußglocke bei einem Langzeitbetrieb der Vorrichtung dem im Wassertopf aufgrund von Verdampfung abnehmenden Wasserstand einwandfrei und betriebssicher nachgeführt werden; denn das Wasser im Wassertopf wird mittels der Beheizeinrichtung auf einer für die Freisetzung ätherischer Wirkstoffe optimalen Temperatur von 80 bis 90 °C gehalten.

Die Führungseinrichtung ist ein im Zentrum des Wassertopfes stehendes Stangenteil, das unten einen Standfuß aufweist, mit welchem es auf dem Boden des Wassertopfes steht. Sowohl das Führungselement der Verschlußglocke als auch das Führungsorgan des Schwimmnapfes sind als an dem Stangenteil frei auf- und abgleitbar geführte Hülsen ausgebildet. Die Hülsen sind dabei so angeordnet und dimensioniert, daß die Hülse des Schwimmnapfes außen an der Hülse der Verschlußglocke geführt ist, wobei die Hülse der Verschlußglocke wiederum an dem Stangenelement geführt ist.

Schwimmnapf und Verschlußglocke sind zylinderförmig und somit runddosenartig ausgebildet, wobei der runddosenartige Schwimmnapf innen auf seinem Boden stehende radiale Stegwände aufweist, die je eine Abteilung zur Aufnahme eines Teils einer Salbenportion begrenzen, die in den Schwimmnapf insgesamt eingegeben werden soll. Selbstverständlich können Schwimmnapf und Verschlußglocke auch andere, von der Kreisform abweichende Formgebungen aufweisen.

Schwimmnapf und Verschlußglocke sind vorzugsweise Spritzgußteile aus Kunststoff. Andere Werkstoffe sind verwendbar, wobei jedoch gewährleistet sein muß, daß die Salbe bzw. die aus ihr freigesetzten Ausdünstungen keinerlei unerwünschte Reaktion mit dem Wirkstoff eingehen.

Ein Ausführungsbeispiel der Erfindung, aus dem sich weitere erfinderische Merkmale ergeben, ist in der Zeichnung dargestellt. Es zeigen:
- Fig. 1: eine Seitenansicht einer betriebsbereiten Vorrichtung im Längsschnitt,
- Fig. 2: die Verschlußglocke in der Untenansicht,
- Fig. 3: die Seitenansicht der Verschlußglocke gem. Fig.2 im Schnitt,
- Fig. 4: die Seitenansicht eines Schwimmnapfes im Schnitt und
- Fig. 5: den Schwimmnapf gem. Fig.4 in der Draufsicht.

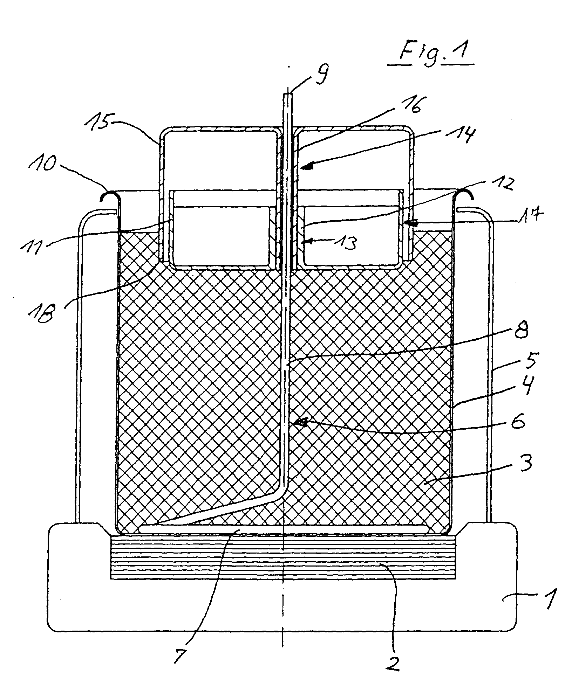

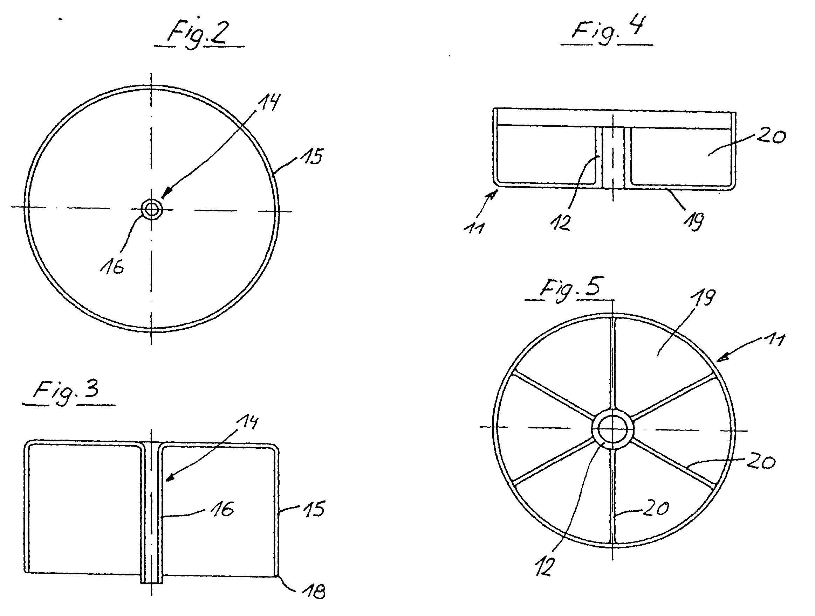

In Fig.1 ist eine betriebsbereite Vorrichtung in längsgeschnittener Seitenansicht dargestellt. Auf einer elektrischen Beheizeinrichtung 1 mit einer Heizplatte 2 steht ein mit Wasser 3, vorzugsweise kochendem Wasser, weitgehend gefüllter Wassertopf 4. Mit 5 ist eine isolierende Außenhaube, z.B. aus Kunststoff, bezeichnet. In den Wassertopf 4 ist eine Führungseinrichtung 6 gestellt, die hier aus einem Metalldraht gebogen ist und die ein Fußteil 7 sowie ein Stangenelement 8 umfaßt. Das Stangenelement 8 ist so bemessen, daß es mit seinem oberen freien Ende 9 über den Öffnungsrand 10 des Wassertopfes 4 hinausragt. Auf dem Wasser 3 schwimmt ein Schwimmnapf 11, in den eine vorbestimmte Menge Salbe eingebbar ist. Der Boden des Schwimmnapfes 11 ist im Zentrum zu einer in das Innere des Schwimmnapfes vorstehenden Hülse 12 hochgezogen. Die Hülse 12 bildet ein Führungsorgan 13, das mit der Führungseinrichtung 6 in Wirkverbindung steht. Dies wird erreicht durch ein Führungselement 14 einer über den Schwimmnapf 11 gestülpten Verschlußglocke 15. Das Führungselement 14 ist ebenfalls als im Zentrum der Verschlußglocke 15 befindlichen Hülse 16 ausgebildet. Die Hülse 16 ist so bemessen, daß auf ihr die Hülse 12 des Schwimmnapfes 11 geführt wird. Gleichzeitig ist die Hülse 16 der Verschlußglocke dafür vorgesehen, um auf dem Stangenteil 8 der Führungseinrichtung 6 geführt zu werden.

Wie Fig. 1 verdeutlicht, ist die Verschlußglocke 15 um ein vorbestimmtes Maß weiter als der Schwimmnapf 11. Dadurch ist zwischen den beiden auf dem Stangenelement zentrisch gehaltenen schwimmenden Teilen ein Spalt oder Zwischenraum 17 gegeben, der, da der untere Glockenrand 18 bei auf dem Wasser schwimmendem Schwimmnapf in das Wasser 3 eingetaucht ist, eine Wasseroberfläche begrenzt, deren Größe durch die Weite des Zwischenraumes 17 bestimmt ist.

Über die Wasseroberfläche im Zwischenraum 17 treten Bestandteile der aus der Salbe im Schwimmnapf freigesetzten Dämpfe in das Wasser 3 ein. Bestimmte Bestandteil der Dämpfe, nämlich die medizinisch wirksamen Bestandteile aus den Wirkstoffen der Salbe, werden über die freie Oberfläche des Wassers 3 im Wassertopf 1 dann an die Außenluft der Umgebung der Vorrichtung freigesetzt.

In Fig.2 ist eine Untenansicht einer Verschlußglocke 15 dargestellt, die die Form eines zylinderförmigen Topfes hat. Im Zentrum befindet sich ein Loch, das in ein in die Verschlußglocke 15 vorstehendes Führungselement 14 übergeht.

Wie die in Fig.3 dargestellte Seitenansicht der Verschlußglocke 15 verdeutlicht, steht das freie Ende des hier als Hülse 16 vorliegenden Führungselementes 14 über den unteren Glockenrand 18 vor.

Fig.4 zeigt eine Seitenansicht eines Schwimmnapfes 11 im Schnitt. Gleiche Bauteile sind mit gleichen Bezugszahlen wie in Fig. 1 bezeichnet.

Erkennbar und in Fig.5, die eine Draufsicht des Schwimmnapfes 11 zeigt, weiter verdeutlicht, sind die auf dem Boden 19 des Schwimmnapfes 11 stehenden radialen Stegwände 20, die im Inneren der Schwimmwanne 11 befindliche Abteile begrenzen, in welche jeweils vorbestimmte Mengen einer Salbe eingebbar sind. Die Abteile erlauben eine Dosierung der Salbe, denn die Menge der Salbe und somit die Quantität der abgedunsteten Wirkstoffe ist umso höher, je mehr Abteile mit Salbe gefüllt werden.

## Patentansprüche

1. Vorrichtung zur Freisetzung ätherischer Wirkstoffe aus Heilsalben mit einem Wassertopf, einer Beheizeinrichtung für das im Wassertopf aufgenommene Wasser, mit einem eine vorbestimmte Menge der Heilsalbe aufnehmenden, auf der freien Oberfläche des Wassers im Wassertopf schwimmenden Schwimmnapf, und mit einer Führungseinrichtung zum Nachrühren des Schwimmnapfes bei abnehmendem Wasserstand im Wassertopf,
**dadurch gekennzeichnet,**
**daß** eine über den Schwimmnapf (11) stülpbare Verschlußglocke (15) vorgesehen ist, die um ein vorbestimmtes Maß weiter als der übergriffene Schwimmnapf (11) ist und deren unterer Glockenrand (18) bei auf dem Wasser (3) schwimmendem Schwimmnapf (11) in das Wasser (3) eingetaucht ist.

2. Vorrichtung nach Anspruch 1, **dadurch gekennzeichnet, daß** die Verschlußglocke (15) ein mit der Führungseinrichtung (6) in Wirkverbindung bringbares Führungselement (14) aufweist.

3. Vorrichtung nach einem der Ansprüche 1 und 2, **dadurch gekennzeichnet, daß** der Schwimmnapf (11) ein mit der Führungseinrichtung (6) in Wirkverbindung bringbares Führungsorgan (13) aufweist.

4. Vorrichtung nach einem der vorhergehenden Ansprüche, **dadurch gekennzeichnet, daß** die Führungseinrichtung (6) ein im Zentrum des Wassertopfes (4) stehendes, über den freien Öffnungsrand (10) des Wassertopfes (4) und damit über die Wasseroberfläche ragendes Stangenelement (8) aufweist.

5. Vorrichtung nach einem der Ansprüche 1 bis 4, **dadurch gekennzeichnet, daß** Führungsorgan (13) und Führungselement (14) an dem Stangenelement (8) auf- und abgleitbar geführte Hülsen (12, 16) sind.

6. Vorrichtung nach Anspruch 5, **dadurch gekennzeichnet, daß** die Hülsen (12, 16) so dimensioniert sind, **daß** die Hülse (12) des Schwimmnapfes (11) außen an der Hülse (16) der Verschlußglocke (15) geführt ist, wobei die Hülse (16) der Verschlußglocke (15) wiederum an dem Stangenelement (8) geführt ist.

7. Vorrichtung nach einem der vorhergehenden Ansprüche, **dadurch gekennzeichnet, daß** der Schwimmnapf (11) Abteilungen für einzelne Salbenportionen aufweist.

8. Vorrichtung nach einem der vorhergehenden Ansprüche, **dadurch gekennzeichnet, daß** der Schwimmnapf (11) und die Verschlußglocke (15) zylinderförmig ausgebildet sind.

9. Vorrichtung nach Anspruch 8, **dadurch gekennzeichnet, daß** der Schwimmnapf (11) auf seinem Boden (19) stehende Stegwände (20) aufweist, die je eine Abeilung begrenzen.

10. Vorrichtung nach einem der vorhergehenden Ansprüche, **dadurch gekennzeichnet, daß** Schwimmnapf (11) und Verschlußglocke (15) Spritzgußteile aus Kunststoff sind.
